# EUROPÄISCHE PATENTSCHRIFT

(11) **EP 0 853 494 B1**
(45) Veröffentlichungstag und Bekanntmachung des Hinweises auf die Patenterteilung: **24.04.2002**
(21) Anmeldenummer: 96930995.4
(22) Anmeldetag: 02.09.1996
(51) Int. Cl.: B01F 17/00

(54) **O/W-EMULGATOREN**
OIL-WATER EMULSIFIERS
EMULSIFIANTS HUILE DANS EAU

(30) Priorität: 11.09.1995 DE 19533539
(43) Veröffentlichungstag der Anmeldung: 22.07.1998
(73) Patentinhaber: Cognis Deutschland GmbH & Co. KG, 40589 Düsseldorf (DE)
(72) Erfinder: ANSMANN, Achim, D-40699 Erkrath (DE); KAWA, Rolf, D-40789 Monheim (DE); GONDEK, Helga, D-40589 Düsseldorf (DE); KOESTER, Josef, D-40221 Düsseldorf (DE); SCHWARZ, Annette, D-40229 Düsseldorf (DE)
(86) Internationale Anmeldenummer: EP9603837
(87) Internationale Veröffentlichungsnummer: WO9710049

(56) Entgegenhaltungen:
- WO-A-95/14519
- WO-A-95/34528
- DE-A- 4 229 442
- DE-A- 4 414 815
- DATABASE WPI Week 9233, Derwent Publications Ltd., London, GB; AN 1992-272108 'Emulsified cosmetic material' & JP 04 178 316 A (KAO CORP) 25 Juni 1992

## Beschreibung

### Gebiet der Erfindung

Die Erfindung betrifft neue O/W-Emulgatoren, enthaltend ausgewählte Tenside in Kombination mit bestimmten Polymeren sowie deren Verwendung zur Herstellung von kosmetischen bzw. pharmazeutischen Zubereitungen.

### Stand der Technik

Polyglycerinpolyricinoleate sind seit langem als Emulgatoren bekannt und können zur Formulierung von niedrigviskosen Emulsionen eingesetzt werden [vgl. **EP-A1 0559013** (Th. Goldschmidt), **EP-A1 0440203** (Lotte Co.) und **WO 85/04346** (Meiji Milk Prods.)]. Es zeigt sich jedoch, daß Polyglycerinpolyrinoleate des Marktes nicht mit allen in der Kosmetik üblicherweise eingesetzten Öle Emulsionen bilden, sondern nur mit solchen eines bestimmten Polaritätsbereiches; zudem sind diese Emulsionen nur eingeschränkt lagerstabil. Ein wesentlicher Nachteil besteht vor allem darin, daß die handelsüblichen Produkte nicht in der Lage sind, Emulsionen mit stark polaren Ölen wie beispielsweise Pflanzenölen ausreichend zu stabilisieren. Im Hinblick auf die besondere ökotoxikologische Verträglichkeit derartiger Emulsionen wird jedoch gerade dies im Markt gewünscht.

Aus der Europäischen Patentschrift **EP-B1 0 553 241** (SEPPIC) sind ebenfalls selbstemulgierende Mischungen, enthaltend Alkylpolyglucoside und Fettalkohole der korrespondierenden Kettenlänge bekannt. Bei Einsatz derartiger Emulgatoren beobachtet man jedoch in vielen Fällen unerwünschte Viskositätsänderungen über die Lagerzeit.

Die Aufgabe der Erfindung hat nun darin bestanden, neue ethylenoxidfreie O/W- Emulgatoren zur Verfügung zu stellen, die mit einem breiten Spektrum von Ölkörpern lagerstabile Emulsionen ergeben.

### Beschreibung der Erfindung

Gegenstand der Erfindung sind O/W-Emulgatoren, enthaltend
a1) Alkyl- und/oder Alkenyloligoglykoside, und/oder
a2) Fettsäure-N-alkylpolyhydroxyalkylamide und/oder
a3) Acylglutamate und
b) Polyolpolyhydroxystearate,
mit der Maßgabe, daß das Gewichtsverhältnis a) : b) im Bereich von 90 : 10 bis 10 : 90, vozugsweise 70 : 30 bis 30 : 70 und insbesondere bei 60 : 40 bis 40 : 60 liegt.

Überraschenderweise wurde gefunden, daß bei Einsatz von Abmischungen der genannten Tenside, vorzugsweise C_{12/14}-Alkyloligoglucosiden, mit Polyolpolyhydroxystearaten, vorzugsweise Polyglycerinpoly-12-hydroxystearaten, als O/W-Emulgatoren Emulsionen erhalten werden, die unabhängig von der Polarität des eingesetzten Ölkörpers auch bei Temperaturschwankungen lagerstabil sind. Die Erfindung schließt die Erkenntnis ein, daß die Mischungen flüssig und leicht pumpbar sind, insbesondere dann, wenn als zusätzliche Komponente mindestens ein Polyol, vorzugsweise Glycerin, mitverwendet wird.

### Alkyl- und/oder Alkenyloligoglykoside

Alkyl- und Alkenyloligoglykoside stellen bekannte nichtionische Tenside dar, die der Formel **(I)** folgen,

**R**^{**1**}**O-[G]**_{**p**} **(I)**

in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die Schriften **EP-A1-0 301 298** und **WO 90/03977** verwiesen.

Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl-und/oder Alkenyloligoglykoside sind somit Alkyl- und/oder Alkenyloligo**glucoside.**

Die Indexzahl p in der allgemeinen Formel **(I)** gibt den Oligomerisierungsgrad (DP-Grad), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt.

Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3).

Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

### Fettsäure-N-alkylpolyhydroxyalkylamide

Fettsäure-N-alkylpolyhydroxyalkylamide stellen nichtionische Tenside dar, die der Formel **(II)** folgen, 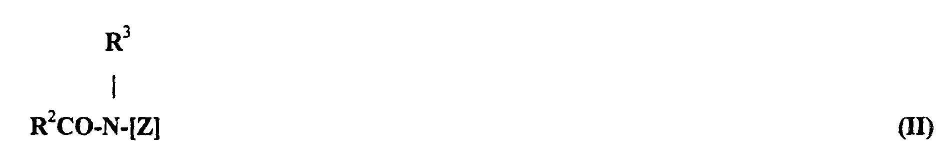 in der R²CO für einen aliphatischen Acylrest mit 6 bis 22 Kohlenstoffatomen, R³ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften **US 1,985,424, US 2,016,962** und **US 2,703,798** sowie die Internationale Patentanmeldung **WO 92/06984** verwiesen. Eine Übersicht zu diesem Thema von H.Kelkenberg findet sich in **Tens. Surf. Det. 25**, **8 (1988).** Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher **Fettsäure-N-alkylglucamide** dar, wie sie durch die Formel **(III)** wiedergegeben werden:

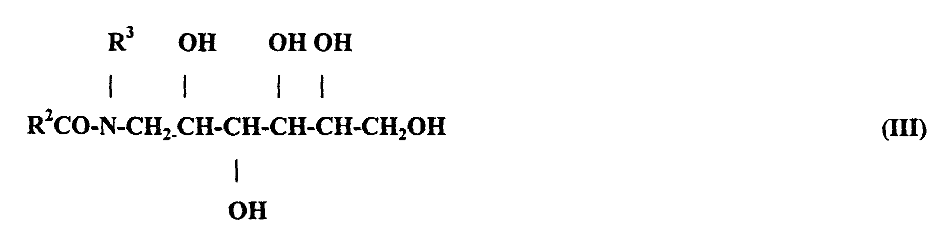

Vorzugsweise werden als Fettsäure-N-alkylpolyhydroxyalkylamide Glucamide der Formel **(III)** eingesetzt, in der R³ für Wasserstoff oder eine Alkylgruppe steht und R²CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkyl-glucamide der Formel **(III)**, die durch reduktive Aminierung von Glucose mit Methylamin und anschließende Acylierung mit Laurinsäure oder C_{12/14}-Kokosfettsäure bzw. einem entsprechenden Derivat erhalten werden. Weiterhin können sich die Polyhydroxyalkylamide auch von Maltose und Palatinose ableiten.

Auch die Verwendung der Fettsäure-N-alkylpolyhydroxyalkylamide ist Gegenstand einer Vielzahl von Veröffentlichungen. Aus der Europäischen Patentanmeldung **EP-A1 0 285 768** (Hüls) ist beispielsweise ihr Einsatz als Verdickungsmittel bekannt. In der Französischen Offenlegungsschrift **FR-A 1 580 491** (Henkel) werden wäßrige Detergensgemische auf Basis von Sulfaten und/oder Sulfonaten, Niotensiden und gegebenenfalls Seifen beschrieben, die Fettsäure-N-alkylglucamide als Schaumregulatoren enthalten. Mischungen von kurz- und längerkettigen Glucamiden werden in der Deutschen Patentschrift **DE-C1 44 00 632** (Henkel) beschrieben. In den Deutschen Offenlegungsschriften **DE-A1 42 36 958** und **DE-A1 43 09 567** (Henkel) wird ferner über den Einsatz von Glucamiden mit längeren Alkylresten als Pseudoceramide in Hautpflegemitteln sowie über Kombinationen von Glucamiden mit Proteinhydrolysaten und kationischen Tensiden in Haarpflegeprodukten berichtet.

Gegenstand der Internationalen Patentanmeldungen **WO 92/06153; WO 92/06156; WO 92/06157; WO 92/06158; WO 92/06159** und **WO 92/06160** (Procter & Gamble) sind Mischungen von Fettsäure-N-alkyl-glucamiden mit anionischen Tensiden, Tensiden mit Sulfatund/oder Sulfonatstruktur, Ethercarbonsäuren, Ethersulfaten, Methylestersulfonaten und nichtionischen Tensiden. Die Verwendung dieser Stoffe in den unterschiedlichsten Wasch-, Spül- und Reinigungsmitteln wird in den Internationalen Patentanmeldungen **WO 92/06152; WO 92/06154; WO 92/06155; WO 92/06161; WO 92/06162; WO 92/06164; WO 92/06170; WO 92/06171** und **WO 92/06172** (Procter & Gamble) beschrieben.

### Acylglutamate

Acylglutamate stellen bekannte anionische Tenside dar, die der Formel **(IV)** folgen, 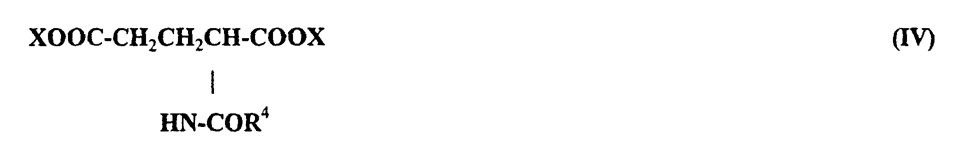 in der R⁴CO für einen linearen oder verzweigten Acylrest mit 6 bis 22 Kohlenstoffatomen und 0 und/oder 1, 2 oder 3 Doppelbindungen und X für Wasserstoff, ein Alkali- und/oder Erdalkalimetall, Ammonium, Alkylammonium, Alkanolammonium oder Glucammonium steht. Ihre Herstellung erfolgt beispielsweise durch Schotten-Baumann Acylierung von Glutaminsäure mit Fettsäuren, Fettsäureestern oder -chloriden. Verkaufsprodukte sind beispielsweise von der Hoechst AG, Frankfurt/DE oder der Ajinomoto Co. Inc., Tokyo/JP erhältlich. Eine Übersicht zu Herstellung und Eigenschaften der Acylglutamate findet sich von M.Takehara et al. in **J. Am. Oil. Chem. Soc., 49, 143 (1972)**. Typische Beispiele für geeignete Acylglutamate, die im Sinne der Erfindung in Betracht kommen, sind Aniontenside, die sich von Fettsäuren mit 6 bis 22, vorzugsweise 12 bis 18 Kohlenstoffatomen ableiten, wie beispielsweise C_{12/14}- bzw. C_{12/18}-Kokosfettsäure, Laurinsäure, Myristinsäure, Palmitinsäure und/oder Stearinsäure. Besonders bevorzugt sind Natrium- bzw. Kalium-N-cocoyl- und Natriumbzw. Kalium-N-stearoyl-L-glutamat.

### Polyolpolyhydroxystearate

Polyolpolyhydroxystearate stellen Ester von Polyolen und Polyhydroxystearinsäuren dar. Die Polyolkomponente kann sich beispielsweise von Glycerin, Ethylenglycol, Diethylenglycol, Propylenglycol, Polyglycerin, Trimethylolethan, Trimethylolpropan, Trimethylolbutan, Pentaerythrit, Dipentaerythrit, Methyl- und Butylglucosid, Sorbit, Mannit, Glucose, Saccharose oder Glucamin ableiten. Entsprechende Stoffe sind beispielsweise aus den Druckschriften **GB-A 15 24 782** oder **EP-A 0 000 424** bekannt.

Vorzugsweise handelt es sich bei den Stoffen der Komponente b) um Polyglycerinpolyhydroxystearate, die man erhält, indem man Polyhydroxystearinsäure mit einem Eigenkondensationsgrad im Bereich von 2 bis 20, vorzugsweise 2 bis 10, mit einem Polyglyceringemisch der bevorzugten Zusammensetzung (GC-Methode)

| | |
|---|---|
| Glycerin | 5 bis 35 (15 bis 30) Gew.-% |
| Diglycerine | 15 bis 40 (20 bis 32) Gew.-% |
| Triglycerine | 10 bis 35 (15 bis 25) Gew.-% |
| Tetraglycerine | 5 bis 20 ( 8 bis 15) Gew.-% |
| Pentaglycerine | 2 bis 10 ( 3 bis 8) Gew.-% |
| Oligoglycerine | ad 100 Gew.-% |

in an sich bekannter Weise verestert; in Klammern angegeben sind die bevorzugten Bereiche. Die Herstellung der Polyolpolyhydroxystearate kann in an sich bekannter Weise erfolgen. Im Fall der Polyglycerinpolyhydroxystearate wird dabei vorzugsweise zunächst das Polyglycerin und dann die Polyhydroxystearinsäure hergestellt und schließlich beide verestert. Die Herstellung eines Polyglycerins der oben genannten Zusammensetzung kann durch Eigenkondensation von Glycerin in Gegenwart von geeigneten Katalysatoren wie beispielsweise Kaliumcarbonat, Silicaten gemäß **DE-A1 40 29 323** (Henkel) oder Boraten gemäß **DE-A1 41 17 033** (Henkel) bei Temperaturen im Bereich von 200 bis 260°C durchgeführt werden. Die Herstellung der Polyhydroxystearinsäure erfolgt beispielsweise durch alkalisch katalysierte Polykondensation von Hydroxystearinsäure, vorzugsweise 12-Hydroxystearinsäure, die durch Härtung von Ricinolsäure bzw. technischer Ricinusölfettsäure gewonnen wird. Vorzugsweise werden dabei lineare Veresterungsprodukte mit 2 bis 10 und insbesondere 2 bis 8 Fettsäureeinheiten gebildet. Typischerweise wird die folgende Verteilung (GPC-Methode) erreicht:

| | |
|---|---|
| Monomere | 1 bis 10 Gew.-% |
| Dimere | 5 bis 15 Gew.-% |
| Trimere | 5 bis 15 Gew.-% |
| Tetramere | 5 bis 15 Gew.-% |
| Pentamere | 5 bis 15 Gew.-% |
| Hexamere | 5 bis 15 Gew.-% |
| Heptamere | 5 bis 15 Gew.-% |
| Octamere | 1 bis 10 Gew.-% |
| Oligomere | ad 100 Gew.-% |

In einer besonderen Ausführungsform der Erfindung werden Gemische von Hydroxystearinsäure und Ricinolsäure bzw. technischer Ricinusölfettsäure, die zu etwa 90 Gew.-% aus Ricinolsäure besteht, im Gewichtsverhältnis 99 : 1 bis 1 : 99 und vorzugsweise 75 : 25 bis 10 : 90 eingesetzt. In gleicher Weise ist es möglich, die Säuren einzeln zu kondensieren und anschließend die Kondensate abzumischen. Bei der nachfolgenden Kondensation der Polyolkomponente, beispielsweise des Polyglycerins mit der Polyhydroxystearinsäure bzw. den Gemischen mit Polyricinolsäure, wird eine komplexe Mischung homologer Polyester gebildet. Die Anteile an Mono-, Di-, Tri- und Oligoestern in den erfindungsgemäßen Polyolpolyhydroxystearaten und vorzugsweise Polyglycerinpolyhydroxystearaten richtet sich nach den Einsatzverhältnissen der Ausgangsverbindungen. In einer bevorzugten Ausführungsform des erfindungsgemäßen Verfahrens wird ein Polyolpolyhydroxystearat mit besonders vorteilhaften anwendungstechnischen Eigenschaften erhalten, indem man etwa 1000 kg 12-Hydroxystearinsäure solange einer Eigenkondensation unterwirft, bis ein Produkt mit einer Säurezahl im Bereich von 50 bis 55 resultiert und dieses dann mit etwa 150 kg Polyglycerin der oben angegebenen Zusammensetzung weiter verestert, bis die Säurezahl bis auf einen Wert kleiner 2 abgenommen hat. Kondensationsprodukte auf Basis von Polyglycerin und Polyhydroxystearinsäure bzw. Polyhydroxystearinsäure/Polyricinolsäure können über ihre Iodzahl charakterisiert werden. Typische Beispiele sind Polyester mit einer Iodzahl < 10 (Basis 100 % 12-Hydroxystearinsäure) bzw 65 bis 80 (Basis 90 % 12-Hydroxystearinsäure, 10 % Ricinolsäure).

### Polyole

Polyole, die im Sinne der Erfindung als weitere Bestandteile der neuen Emulgatoren in Betracht kommen, besitzen 2 bis 15 Kohlenstoffatome und mindestens 2 Hydroxylgruppen. Typische Beispiele sind
- Glycerin;
- Alkylenglycole wie beispielsweise Ethylenglycol, Diethylenglycol, Propylenglycol, Polyethylen- bzw. Polypropylenglycole mit Molmassen im Bereich von 100 bis 1000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin.

Der Anteil der Polyole, vorzugsweise Glycerin, an den neuen O/W-Emulgatoren kann 5 bis 30 und vorzugsweise 15 bis 25 Gew.-% - bezogen auf die Emulgatoren - betzragen.

### Gewerbliche Anwendbarkeit

Die neuen O/W-Emulgatoren eignen sich in besonderer Weise zur Herstellung von temperatur- und lagerstabilen Emulsionen und sind dabei unabhängig von der Polarität der zu emulgierenden Ölkomponente. Insbesondere lassen sich auch unter Verwendung von sehr polaren Ölen rasch und zuverlässig Emulsionen einer zufriedenstellend hohen Viskosität herstellen.

Ein weiterer Gegenstand der Erfindung betrifft die Verwendung der neuen O/W-Emulgatoren zur Herstellung von kosmetischen und/oder pharmazeutischen Zubereitungen wie beispielsweise Cremes, Lotionen, Salben und dergleichen.

Die mit Hilfe der erfindungsgemäßen O/W-Emulgatoren erhältlichen Emulsionen können weitere Hilfs- und Zusatzstoffe wie z.B. Tenside, Ölkörper, Co-Emulgatoren, Überfettungsmittel, Fette, Wachse, Stabilisatoren, biogene Wirkstoffe, Konservierungsmittel, Farbund Duftstoffe enthalten.

Die Emulsionen können mit den anderen Inhaltsstoffen kompatible Tenside enthalten. Typische Beispiele sind Fettalkoholpolyglycolethersulfate, Monoglyceridsulfate, Monound/oder Dialkylsulfosuccinate, Fettsäureisethionate, Fettsäuresarcosinate, Fettsäuretauride, Ethercarbonsäuren, Alkylamidobetaine und/oder pfanzliche Eiweißfettsäurekondensate.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₁₈-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimerdiol) und/oder Guerbetalkoholen, Triglyceride auf Basis C₆-C₁₀-Fettsäuren, pflanzliche Öle, verzweigte primäre Alkohole, substituierte Cyclohexane, Guerbetcarbonate, Dialkylether und/oder aliphatische bzw. naphthenische Kohlenwasserstoffe in Betracht.

Als **Co-Emulgatoren** können nichtionogene, ampholytische und/ oder zwitterionische grenzflächenaktive Verbindungen verwendet werden, die sich durch eine lipophile, bevorzugt lineare, Alkyl- oder Alkenylgruppe und mindestens eine hydrophile Gruppe auszeichnen. Diese hydrophile Gruppe kann sowohl eine ionogene als auch eine nichtionogene Gruppe sein.

Nichtionogene Emulgatoren enthalten als hydrophile Gruppe z. B. eine Polyolgruppe, eine Polyalkylenglycolethergruppe oder eine Kombination aus Polyol- und Polyglycolethergruppe. Bevorzugt sind solche Mittel, die als **O/W-Emulgatoren** nichtionogene Tenside aus mindestens einer der folgenden Gruppen enthalten: (a1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe; (a2) C_{12/18}-Fettsäuremono- und -diester von Anlagerungsprodukten von 1 bis 30 Mol Ethylenoxid an Glycerin; (a3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte; (a4) ethoxylierte Alkylmono- und -oligoglycoside mit 8 bis 22 Kohlenstoffatomen im Alkylrest und (a5) Anlagerungsprodukte von 15 bis 60 Mol Ethylenoxid an Ricinusöl und/oder gehärtetes Ricinusöl. Ebenfalls geeignet sind Gemische von Verbindungen aus mehreren dieser Substanzklassen. Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt. Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyl-dimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxy-ethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin.

Als **Überfettungsmittel** können Substanzen wie beispielsweise polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen. Typische Beispiele für **Fette** sind Glyceride, als **Wachse** kommen u.a. Bienenwachs, Paraffinwachs oder Mikrowachse gegebenenfalls in Kombination mit hydrophilen Wachsen, z.B. Cetylstearylalkohol und/oder Partialglyceride der Palmitin- bzw. Stearinsäure in Frage. Als **Stabilisatoren** können Metallsalze von Fettsäuren wie z.B. Magnesium-, Aluminium und/oder Zinkstearat eingesetzt werden. Unter **biogenen Wirkstoffen** sind beispielsweise Pflanzenextrakte und Vitaminkomplexe zu verstehen. Als **Konservierungsmittel** eignen sich beispielsweise Phenoxyethanol, Formaldehydlösung, Parabene, Pentandiol oder Sorbinsäure. Als **Perlglanzmittel** kommen beispielsweise Glycoldistearinsäureester wie Ethylenglycoldistearat, aber auch Fettsäuremonoglycolester in Betracht. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann in an sich bekannter Weise, d.h. beispielsweise durch Heiß-, Kalt-, Heiß-Heiß/Kalt- bzw. PIT-Emulgierung erfolgen. Hierbei handelt es sich um ein rein mechanisches Verfahren, eine chemische Reaktion findet nicht statt.

### Beispiele

### Beispiele 1 bis 9

Es wurden 9 Beispielrezepturen unter Verwendung einer Mischung aus C_{12/14}-Kokosalkyloligoglucosid (Plantaren® APG 1200, Henkel KGaA, Düsseldorf/FRG) und Polyglycerinpoly-12-hydroxystearat (Dehymuls® PGPH, Henkel KGaA) im Gewichtsverhältnis 60 : 40 (Beispiele R1 bis R5) bzw. 40 : 60 (Beispiele R6 bis R9) hergestellt. Hierzu wurde bei der Kaltherstellung (Verfahren A) die Phase I bei 20°C homogen verrührt, Phase II unter Rühren langsam zugegeben, anschließend Phase III eingerührt, homogenisiert und schließlich Phase IV beigemischt. Im Rahmen der Heißherstellung (Verfahren B) wurde Phase I auf 85°C erwärmt und homogen verrührt, die vorgewärmte Phase II zugegeben und die Emulsion anschließend abgekühlt. Die Zusammensetzung der Emulsionen ist Tabelle 1 zu entnehmen:

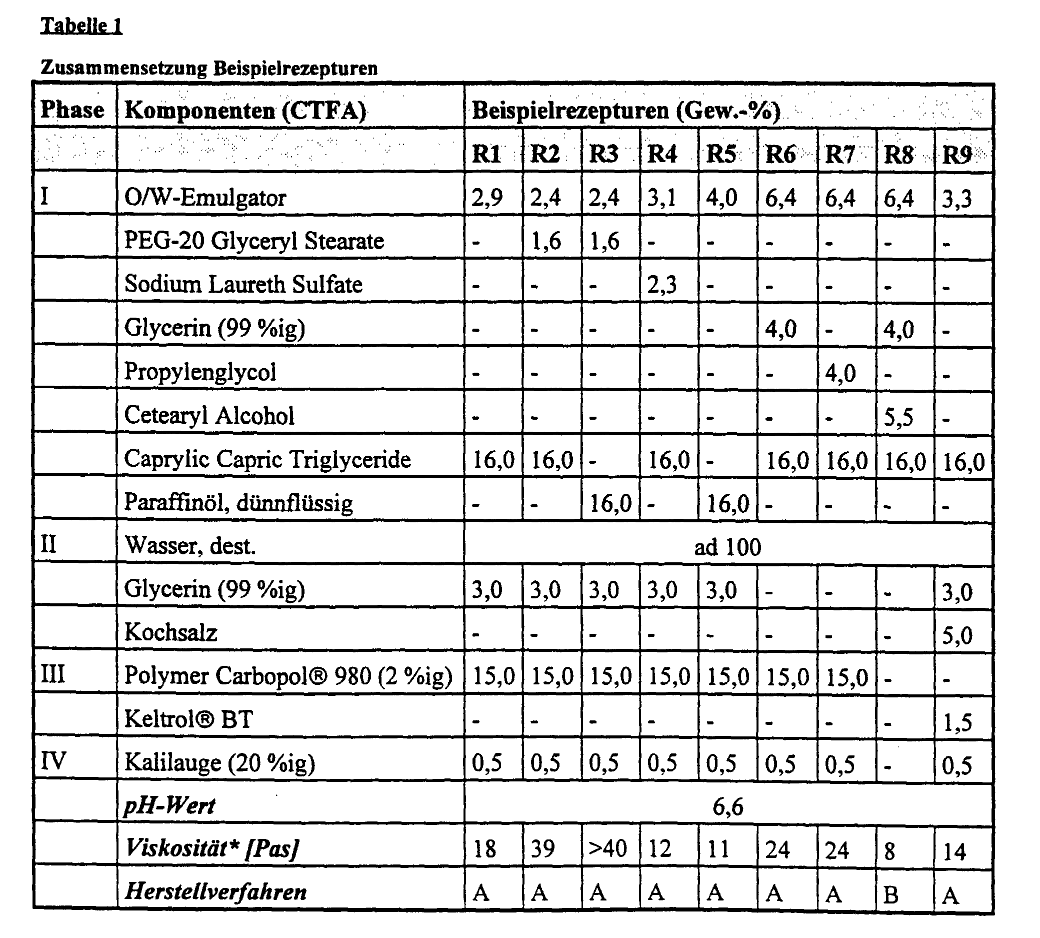

Es wurden lagerstabile Emulsionen erhalten, die eine Viskosität nach Brookfield (RVF, 23°C, 10 UpM, Spindel 5 bzw. 6 in Bsp.R3) im Bereich von 5 bis 50.000 mPas aufwiesen.

### Beispiele 10 bis 13, Vergleichsbeispiele V1 und V2

Es wurden Mischungen enthaltend 20 Gew.-% Mandelöl, 7 Gew.-% Emulgator, 5 Gew.-% Glycerin (86 Gew.-%ig) und 5 Gew.-% Magnesiumsulfat-7-Hydrat (Wasser ad 100 Gew.-%) hergestellt, über einen Zeitraum von 4 Wochen gelagert und die Viskosität bestimmt. Folgende Emulgatoren wurden getestet:
A1) Plantaren® APG 1200/Dehymuls® PGPH = 60 : 40
A2) Plantaren® APG 1200/Dehymuls® PGPH = 40 : 60
A3) Plantaren® APG 1200/Dehymuls® PGPH/Glycerin = 25 : 50 : 25
A4) Laurinsäure-N-methylglucamid/Dehymuls® PGPH = 60 : 40
B1) Polyglycerinpolyricinoleat
B2) Glycerinpoly-12-hydroxystearat

Die Ergebnisse sind in Tabelle 2 zusammengefaßt:

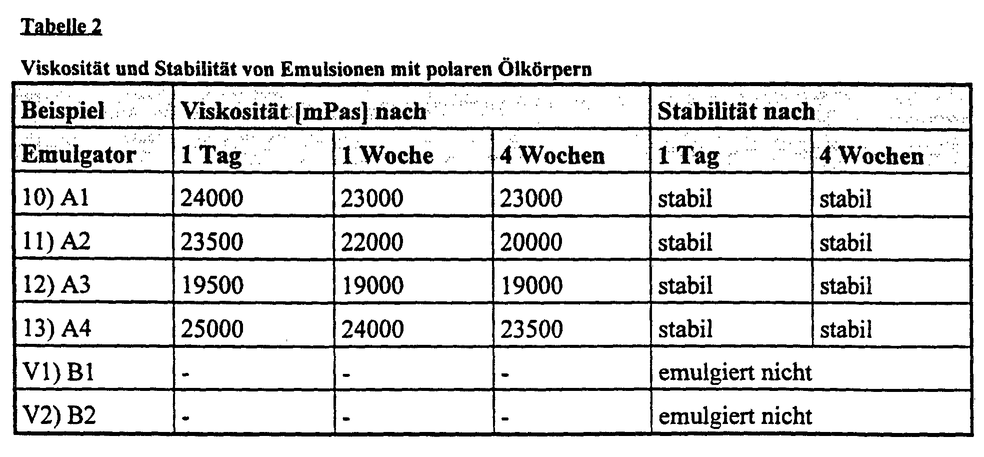

### Beispiele 14 und 15, Vergleichsbeispiele V3 und V4

Nach dem Heißverfahren wurden jeweils zwei Emulsionen unter Verwendung der erfindungsgemäßen Emulgatormischung und einer ethylenoxidhaltigen Vergleichsmischung bestehend aus Ceteareth-12 und Ceteareth-20 hergestellt. Die Viskosität der Emulsionen wurde wie oben nach der Brookfield-Methode bestimmt und über einen Zeitraum von 1 Tag bis 8 Wochen verfolgt. Die Stabilität wurde bei Raumtemperatur bzw. 40°C nach 4 bzs. 8 Wochen beurteilt. Die Rezepturen R10 und R12 sind erfindungsgemäß, die Rezepturen R11 und R13 dienen zum Vergleich. Die Ergebnisse sind in Tabelle 3 zusammengefaßt.

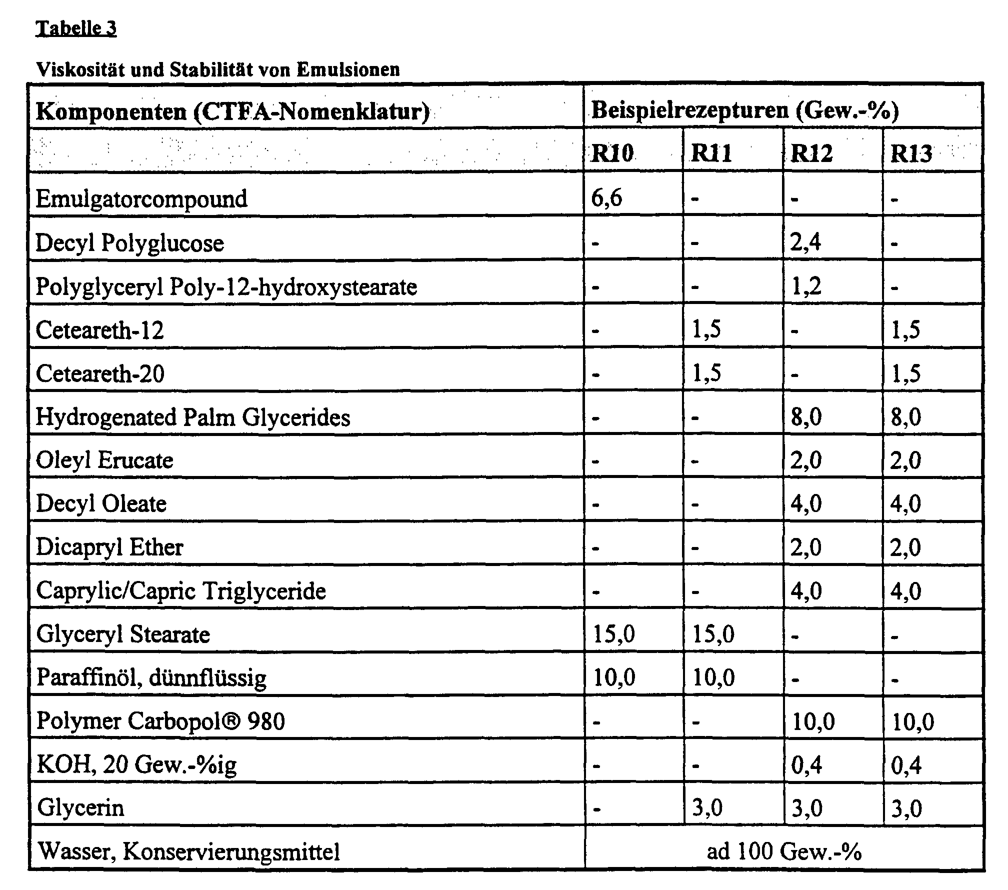

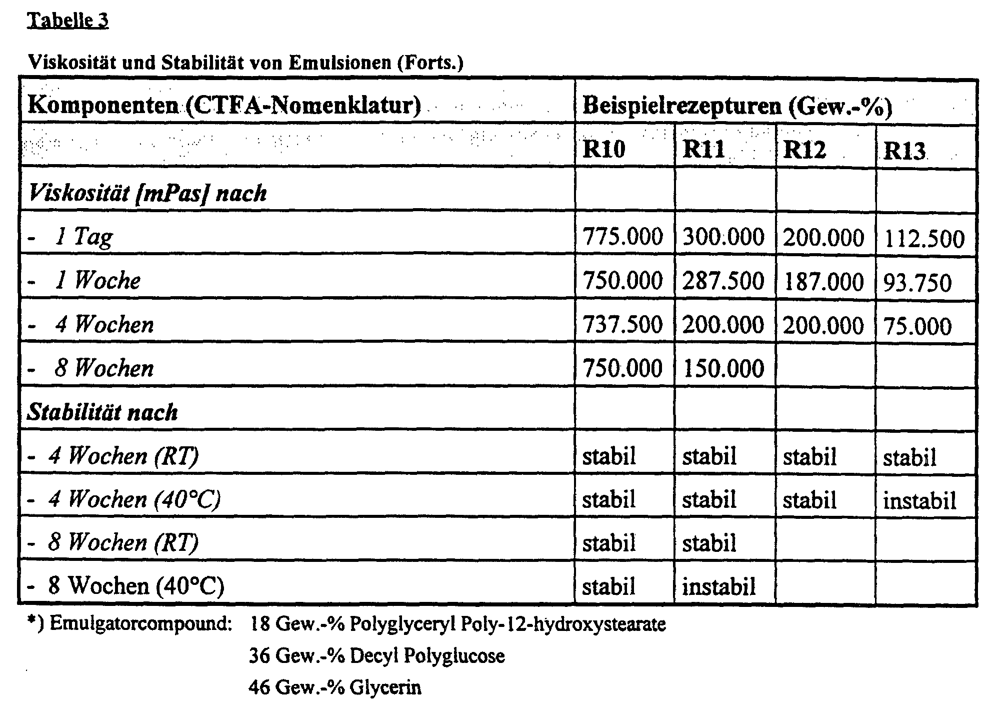

## Patentansprüche

1. O/W-Emulgatoren, enthaltend
a1) Alkyl- und/oder Alkenyloligoglykoside, und/oder
a2) Fettsäure-N-alkylpolyhydroxyalkylamide und/oder
a3) Acylglutamate
b) Polyolpolyhydroxystearate,
mit der Maßgabe, daß das Gewichtsverhältnis a) : b) im Bereich von 90 : 10 bis 10 : 90 liegt.

2. O/W-Emulgatoren nach Anspruch 1, **dadurch gekennzeichnet, daß** sie Alkyl- und/oder Alkenyloligoglykoside der Formel **(I)** enthalten,
**R**^{**1**}**O-[G]**_{**p**} **(I)**
in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht.

3. O/W-Emulgatoren nach den Ansprüchen 1 und 2, **dadurch gekennzeichnet, daß** sie Fettsäure-N-alkylpolyhydroxyalkylamide der Formel **(II)** enthalten, 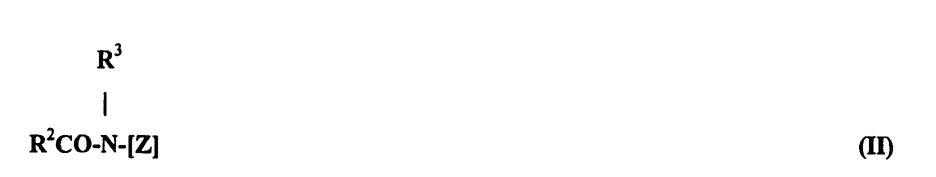 in der R²CO für einen aliphatischen Acylrest mit 6 bis 22 Kohlenstoffatomen, R³ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

4. O/W-Emulgatoren nach den Ansprüchen 1 bis 3, **dadurch gekennzeichnet, daß** sie Acylglutamate der Formel **(IV)** enthalten, 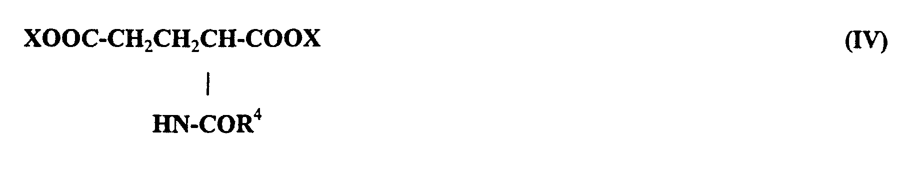 in der R⁴CO für einen linearen oder verzweigten Acylrest mit 6 bis 22 Kohlenstoffatomen und 0 und/ oder 1, 2 oder 3 Doppelbindungen und X für Wasserstoff, ein Alkali- und/oder Erdalkalimetall, Ammonium, Alkylammonium, Alkanolammonium oder Glucammonium steht.

5. O/W-Emulgatoren nach den Ansprüchen 1 bis 4, **dadurch gekennzeichnet, daß** sie Polyglycerinpoly-12-hydroxystearate enthalten.

6. O/W-Emulgatoren nach den Ansprüchen 1 bis 5, **dadurch gekennzeichnet, daß** sie die Zuckertenside der Komponente a) und die Polymeren der Formel b) im Gewichtsverhältnis 70 : 30 bis 30 : 70 enthalten.

7. O/W-Emulgatoren nach den Ansprüchen 1 bis 6, **dadurch gekennzeichnet, daß** sie als weitere Bestandteile Polyole enthalten.

8. O/W-Emulgatoren nach Anspruch 7, **dadurch gekennzeichnet, daß** sie die Polyole in Mengen von 5 bis 30 Gew.-% - bezogen auf die Emulgatoren - enthalten.

9. Verwendung von O/W-Emulgatoren gemäß Anspruch 1 zur Herstellung von kosmetischen und/oder pharmazeutischen Zubereitungen.

## Claims

1. O/W emulsifiers containing
a1) alkyl and/or alkenyl oligoglycosides and/or
a2) fatty acid-N-alkyl polyhydroxyalkylamides and/or
a3) acyl glutamates
b) polyol polyhydroxystearates,
with the proviso that the ratio by weight of a) to b) is in the range from 90:10 to 10:90.

2. O/W emulsifiers as claimed in claim 1, **characterized in that** they contain alkyl and/or alkenyl oligoglycosides corresponding to formula **(I)**:
**R**^{**1**}**O-[G]**_{**p**} **(I)**
where R¹ is an alkyl and/or alkenyl group containing 4 to 22 carbon atoms, G is a sugar unit containing 5 or 6 carbon atoms and p is a number of 1 to 10.

3. O/W emulsifiers as claimed in claims 1 and 2, **characterized in that** they contain fatty acid-N-alkyl polyhydroxyalkylamides corresponding to formula **(II):** 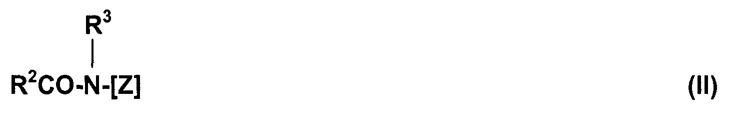 where R²CO is an aliphatic acyl group containing 6 to 22 carbon atoms, R³ is hydrogen, an alkyl or hydroxyalkyl group containing 1 to 4 carbon atoms and [Z] is a linear or branched polyhydroxyalkyl group containing 3 to 12 carbon atoms and 3 to 10 hydroxyl groups.

4. O/W emulsifiers as claimed in claims 1 to 3, **characterized in that** they contain acyl glutamates corresponding to formula **(IV):** 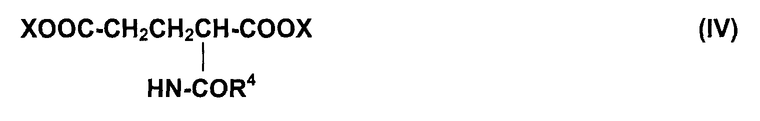 in which R⁴CO is a linear or branched acyl group containing 6 to 22 carbon atoms and 0 and/or 1, 2 or 3 double bonds and X is hydrogen, an alkali metal and/or alkaline earth metal, ammonium, alkylammonium, alkanolammonium or glucammonium.

5. O/W emulsifiers as claimed in claims 1 to 4, **characterized in that** they contain polyglycerol poly-12-hydroxystearates.

6. O/W emulsifiers as claimed in claims 1 to 5, **characterized in that** they contain the sugar surfactants of component a) and the polymers of formula b) in a ratio by weight of 70:30 to 30:70.

7. O/W emulsifiers as claimed in claims 1 to 6, **characterized in that** they contain polyols as further constituents.

8. O/W emulsifiers as claimed in claim 7, **characterized in that** they contain the polyols in quantities of 5 to 30% by weight, based on the emulsifiers.

9. The use of the o/w emulsifiers claimed in claim 1 for the production of cosmetic and/or pharmaceutical formulations.

## Revendications

1. Agents émulsionnants O/W contenant
a1) des alkyl-et/ou alkényloligoglycosides et/ou
a2) des N-alkylpolyhydroxyalkylamides d'acide gras et/ou
a3) des acylglutamates
b) des polyhydroxystéarates de polyol avec la précision que le rapport en poids a) ; b) se situe dans la zone de 90 : 10 à 10 : 90.

2. Agents émulsionnants O/W selon la revendication 1,
**caractérisés en ce qu'**
ils renferment des alkyl- et/ou alkényloligoglycosides de formule (I)
R¹O-[G]ₚ (I)
dans laquelle R¹ représente un reste alkyle et/ou alkényle ayant de 4 à 22 atomes de carbone, G représente un reste de sucre ayant 5 ou 6 atomes de carbone et p représente des nombres allant de 1 à 10.

3. Agents émulsionnants O/W selon les revendications 1 et 2,
**caractérisés en ce qu'**
ils renferment des N-alkylpolyhydroxyalkylamides d'acide gras de formule (II) 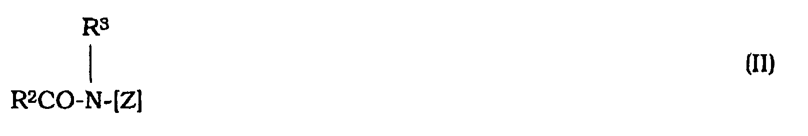 dans laquelle R²CO représente un reste acyle aliphatique ayant de 6 à 22 atomes de carbone, R³ représente de l'hydrogène, un reste alkyle ou un reste hydroxyalkyle ayant de 1 à 4 atomes de carbone et [Z] représente un reste polyhydroxyalkyle linéaire ou ramifié, ayant de 3 à 12 atomes de carbone et de 3 à 10 groupes hydroxyle.

4. Agents émulsionnants O/W selon les revendications 1 à 3,
**caractérisé en ce qu'**
ils renferment des acylglutamates de formule (IV) 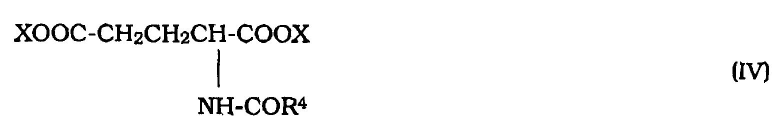 dans laquelle R⁴CO représente un reste acyle linéaire ou ramifié, ayant de 6 à 22 atomes de carbone, et 0 et/ou 1, 2 ou 3 double liaisons et X représente de l'hydrogène, un métal alcalin et/ou un métal alcalinoterreux, de l'ammonium, un alkylammonium, un alkanolammonium ou un glucammonium.

5. Agents émulsionnants O/W selon les revendications 1 à 4,
**caractérisés en ce qu'**
ils renferment des poly-12-hydmstéarates de polyglycérol.

6. Agents émulsionnants O/W selon les revendications 1 à 5,
**caractérisés en ce qu'**
ils renferment des agents tensioactifs dérivés de sucre du composant a) et les polymères de formule b), dans un rapport en poids allant de 70 : 30 à 30 : 70.

7. Agents émulsionnants O/W selon les revendications 1 à 6,
**caractérisés en ce qu'**
ils renferment comme autres constituants des polyols.

8. Agents émulsionnants O/W selon la revendication 7,
**caractérisés en ce qu'**
ils renferment les polyols en quantités allant de 5 à 30 % en poids rapporté aux agents émulsionnants.

9. Utilisation des agents émulsionnants O/W conformément à la revendication 1, pour la production de préparations cosmétiques et/ou pharmaceutiques.
